# EUROPEAN PATENT APPLICATION

(11) **EP 0 775 494 A1**
(43) Date of publication of application: **28.05.1997**
(21) Application number: 95927978.7
(22) Date of filing: 04.08.1995
(51) Int. Cl.: A61K 39/00

(54) **PERORAL IMMUNOGEN COMPOSITION AND PROCESS FOR PRODUCING THE SAME**

(30) Priority: 09.08.1994 JP 187586/94
(71) Applicant: THE NISSHIN OIL MILLS, LTD., Tokyo, 104 (JP)
(72) Inventor: OOYAMA, Katsuhiko, Yokohama-shi Kanagawa 236 (JP); SEKI, Kuniharu, Yokohama-shi Kanagawa 240 (JP); UMEMURA, Masashi, Yokohama-shi Kanagawa 233 (JP); KASAI, Michio, Yokohama-shi Kanagawa 240 (JP); CHIBA, Mitsuru The Nisshin Oil Mills, Ltd., Yokohama-shi Kanawagawa 221 (JP); SHINOHARA, Hisami, Yokohama-shi Kanawagawa 226 (JP); SAGAE, Yoko, Yokohama-shi Kanagawa 240 (JP); NEROME, Kuniaki, Kamakura-shi Kanagawa 248 (JP)
(74) Representative: Plougmann, Vingtoft & Partners A/S
(86) International application number: JP9501562
(87) International publication number: WO9604931

(57) **Abstract**

The invention provides an immunogen composition for oral administration comprising an immunogen and one or more fatty acid esters of sucrose wherein the fatty acid has 6 to 18 carbons.

## Description

The present invention relates to a novel immunogen composition for oral administration and a process for preparing the same.

### PRIOR ARTS

Immunogens capable of immunizing human beings and animals are usually suspended in a buffered saline for administration. Such a suspension is referred to as a vaccine. Known vaccines include inactivated vaccines such as a typhoid vaccine, a pertussis vaccine and a rabies vaccine, attenuated vaccines such as a pathogenic micro-organism vaccine and a live polio vaccine, and toxoids such as a diphtheria toxoid and a tetanus toxoid.

Vaccines are usually administered by injection, especially by hypodermic injection. They are not only inconvenient in administration, but also liable to cause side effects such as redness, fever, amyotrophy in the administered region and shock.

In order to overcome the above drawbacks, vaccines for oral administration have been proposed. However, when vaccines now in use are orally administered, immunogens in the vaccines are inactivated by gastric juice, and accordingly no immunological response takes place. For avoiding the inactivation by gastric juice, vaccines are included in microspheres, liposomes or the like.

Such preparation by use of microspheres or liposomes can avoid effect of gastric juice, but is affected by digestion by digestive enzymes such as lipase and protease in the small intestine, by immune exclusion by secretory IgA [see Nature, 255:745-746 (1975)], and by elimination of foreign bodies by mucin in the small intestine. Therefore, a large amount of immunogen is required for obtaining sufficient immunological response. For example, when an influenza vaccine is administered by hypodermic injection to a human being, a dose per injection is about 15µg in terms of the immunogen. On the other hand, a study on preparation technique using microspheres [Proteinoid Microsphere, Pharmaceutical Research, Vol.10, No.8, p.1243 (1993)] has reported that about 1000µg of the immunogen in two oral administrations are required for inducing the immunological response in a rat; and another study on preparation technique using liposomes [Japanese Unexamined Patent Publication (Kokai) No. Hei 5(1993)-339169] has reported that about 2000µg of the immunogen in five oral administrations are required for inducing the immunological response in a rat.

Further, a report (WO93/19781) on a vaccine enclosed in liposome (NISV) using a non-ionic surfactant (glycerol ester or glycerol ether), though the vaccine is not influenza vaccine but bovine seralbumin (BSA) actually, has reported that, when this preparation is orally administered, 740µg of BSA in two oral administrations are required. This necessary dosage is extremely high as well as the necessary dosages in the aforesaid studies, at present.

Further, in a process of microsphering a vaccine or including a vaccine in liposomes, some of the immunogen of the vaccine remains unmicrosphered or unincluded. This results in loss of the immunogen, which is another problem of the preparation using microspheres or liposomes.

Thus, whichever technique may be used, a dosage of the antigen nearly 100 times higher than the dosage for hypodermic administration to a human being is required for oral administration even to a small animal such as a mouse or a rat. Such a high dosage cannot be considered to be practical. Therefore, there is a high demand for a vaccine for safe, simple oral administration. In other words, there is a high demand for an immunogen composition which is capable of being orally administered to carry the immunogen efficiently to immunocompetent tissue in the alimentary canal, whereby the dosage of the immunogen can be reduced to a far more practical level (preferably the dosage being 100µg or less in a single administration to a mouse) than required by the conventional preparations.

In another conventional technique, an fatty acid ester of sucrose is used as an accelerator for absorption of interferon in a composition for nasal administration [Japanese Patent Publication (Kokoku) No. Hei 6(1994)-51642)]. However, the nasal administration is quite different from oral administration in absorption site, mechanism of absorption, mechanism of action and expression of effects. Moreover, this publication has no description about the effect of the fatty acid ester of sucrose used in a vaccine composition for oral administration.

No vaccine compositions for oral administration has been known so far which are capable of providing vaccine effect efficiently without being affected in the small intestine.

The present invention provides a novel immunogen composition for oral administration comprising an immunogen and an fatty acid ester of sucrose wherein the fatty acid has 6 to 18 carbons.

The novel immunogen composition of the present invention is an orally administered vaccine which is free from the problems of the conventional orally administered vaccines. That is to say, the novel immunogen composition of the present invention is safe and convenient to administer and has vaccinal effect at a practical dosage in terms of immunogen.

In another aspect, the present invention provides a process for preparing an immunogen composition for oral administration, comprising mixing and dissolving an fatty acid ester of sucrose in a vaccine containing an immunogen at a mix ratio by weight of the fatty acid ester of sucrose to the vaccine of 250 to 8000 : 1 and then drying by dehydration to obtain a powdery vaccine. Thus the process of the present invention can eliminate the drawback of the loss of immunogens often seen in the conventional preparation using microspheres, liposomes and the like.

The immunogen of the immunogen composition for oral administration of the present invention, also referred to as antigen, designates a substance which causes production of antibodies and/or sensitized lympocytes in human beings or animals and thereby induces humoral immunity and cellular immunity. The immunogens are not particularly limited providing they can be used for prevention of infectious diseases, including various vaccines. Examples of vaccines are inactivated vaccines such as an influenza vaccine, a Japanese encephalitis vaccines, a pertussis vaccine, a DPT (diphtheria, pertussis and tetanus) vaccine, a cholera vaccine and pneumococcus vaccine, attenuated vaccines such as a polio vaccine, a measles vaccine, a mumps vaccine, a BCG vaccine, a typhus vaccine and a variola vaccine, toxoids such as a diphtheria vaccine, a tetanus vaccine, a trimeresurus toxoid, a typhoid toxoid and a botulinus vaccine, and component vaccines such as an influenza HA vaccine, a hepatitis B vaccine, a hepatitis non-A non-B vaccine, a herpes vaccine, an AIDS vaccine and a cancer vaccine.

The fatty acid ester of sucrose designates an fatty acid ester of sucrose wherein the fatty acid has 6 to 18 carbons and optionally has a double bond. Examples of the fatty acid ester of sucrose include fatty acid monoester of sucrose such as sucrose caproate, sucrose caprylate, sucrose caprate, sucrose laurate, sucrose myristate, sucrose palmitate, sucrose stearate, sucrose palmitoleate, sucrose oleate, sucrose linoleate and sucrose linolenate, among which sucrose laurate is preferred. The fatty acid ester of sucrose has various substitution products from mono-substitution products (monoesters) to octa-substitution products (octaesters). In addition, the mono-substitution product, for example, has isomers as hydroxy groups of sucrose take several positions, so that an extremely large number of different fatty acid esters of sucrose can be conceived. In the present invention, the fatty acid esters of sucrose include all such substitution products and isomers large in number. Further, the fatty acid ester of sucrose may contain about 30% or less of di- or tri-esters and may be a mixture of two or more kinds of fatty acid esters of sucrose. Further, since the fatty acid ester of sucrose desirably has a low moisture absorption in powdery state and a high solubility in water, a vehicle or a solubilizing agent may be added for the purpose of lowering the moisture absorption and enhancing the solubility in water. Examples of the vehicle include celluloses such as hydroxypropyl cellulose (HPC), hydroxypropylmethyl cellulose (HPMC), carboxymethyl cellulose (CMC), carboxymethyl cellulose calcium (CMC-Ca), carboxymethyl cellulose sodium (CMC-Na) and cross-carmelose sodium; a water soluble polymer such as polyethylene glycol (PEG), polyvinyl pyrrolidone (PVP) and cross-povidone; starches such as corn starch, potato starch, wheat starch, alpha starch and partial alpha starch; sugars such as lactose, glucose, mannitol, sucrose and sorbitol; polysaccharides such as pullulan, dextran, dextrin and cyclodextrin. Among them, lactose is preferred as the vehicle.

Preferably, in the immunogen composition of the present invention, the immunogen and the fatty acid ester of sucrose are mixed at a mix weight ratio of 1:250 to 1:8000. The mixing may be carried out by a known method, for example, by dissolving or suspending the fatty acid ester of sucrose in a solution containing the immunogen, followed by drying by dehydration of the mixture and powdering.

As the immunogen, a liquid vaccine and a freeze-dried vaccine may be used after being dissolved in phosphate buffer, saline or purified water. The drying by dehydration is preferably performed at a temperature of 50°C or below with the view to avoiding the denaturalizing of proteins.

The powdery immunogen composition thus obtained is preferably made into an enteric preparation as an orally administerable vaccine in order to evade the deactivation by gastric juice. For the enteric preparation, the powdery immunogen composition may be coated directly with an enteric film, or the powdery immunogen composition may be first prepared into granules, tablets, pills, hard gelatin capsules, soft elastic capsules or microcapsules and then coated with an enteric film. The powdery or granular immunogen composition may also be first coated with an enteric film, and then enclosed in hard gelatic capsules, soft elastic capsules or microcapsules. When the obtained immunogen composition is in the form of suspension or emulsion, such suspension or emulsion of the immunogen composition may be enclosed in hard gelatic capsules, soft elastic capsules, or microcapsules already coated with an enteric film, or alternatively may be enclosed in hard gelatic capsules, soft elastic capsules, or microcapsules, followed by coating with an enteric film.

The above-mentioned preparation can be carried out by a method conventionally used in the art.

At the preparation of the immunogen composition into the above-mentioned preparations, it is possible to add a pharmaceutically acceptable vehicle such as saccharides (e.g., lactose, glucose), polysaccharides (e.g., pullulan, dextrin) and starchs (e.g., corn starch); a pharmaceutically acceptable binder such as hydroxypropyl cellulose (HPC), methyl cellulose (MC), polyvinyl pyrrolidone (PVP), polyvinyl alcohol (PVA) and hydroxypropylmethyl cellulose (HPMC); a pharmaceutically acceptable disintegrator such as crystalline cellulose, carboxymethyl cellulose (CMC), partial α-starch, carboxymethyl cellulose sodium (CMC-Na); a pharmaceutically acceptable lubricant such as magnesium stearate and talc; a pharmaceutically acceptable suspending agent such as tragacanth, acacia gum, xanthan gum and sodium alginate.

The enteric agent used for the enteric coating may suitably be chosen from those known in the art. Apt examples thereof are methacrylic acid-methacrylic acid alkyl ester copopymer, hydroxypropylmethyl cellulose phthalate, hydroxypropyl methyl cellulose acetate succinate, carboxymethylethyl cellulose, cellulose acetate phthalate, purified shellac, white shellac, ethyl cellulose, aminoalkylmethacrylate polymer. The coating may be carried out by a known method such as film coating methods using a fluidized granulator (Flow Coater®), a roll-stirring fluidized granulator (spiral flow coater), a PAN coater (high coater) and a centrifugal fluidized glanule coater (CF).

Preferably, a single clinical dose of the immunogen composition of the present invention for oral administration, though it depends on immunogens used, is usually 12.5 to 10,000µg in terms of immunogen for an adult. The dose also depends on the amount of a fatty acid ester of sucrose mixed, the number of immunization and the age of an subject. For example, in the case of using influenza as immunogen, the composition is orally administered to an adult in usual at such a single dose as contains 12.5µg to 10,000µg of influenza. Then, when no rise in antibody titer is observed, the composition may be administered again two to four weeks after the first administration. However, when the amount of the immunogen is small (12.5 to 100µg), it is preferred that the weight ratio of the fatty acid ester of sucrose with respect to 1 of the immunogen is 1000 or more for one immunization and 500 or more for two immunizations.

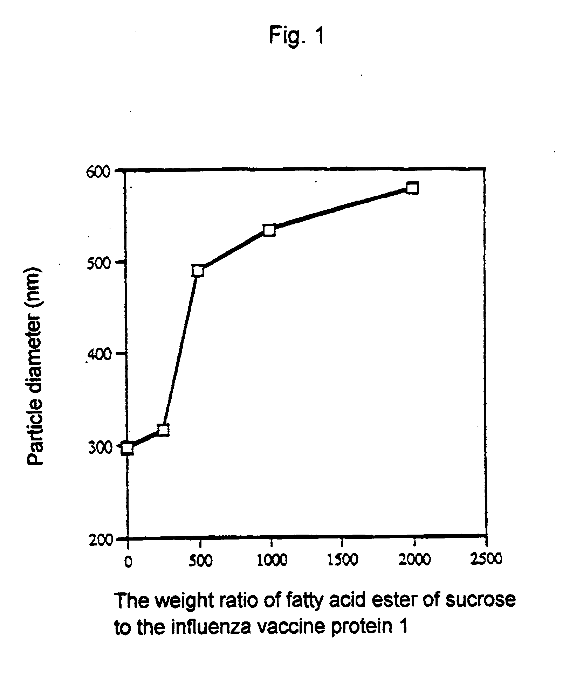

In the immunogen composition of the present invention, it has been found that the average particle diameter of the immunogen is increased by the mixing of the fatty acid ester of sucrose therewith. For example, the average particle diameter of the immunogen in influenza vaccine compositions will be explained. Fig. 1 shows a graph representing the relation between the average particle diameter of the immunogen and the weight ratio of the fatty acid ester of sucrose to the immunogen in the immunogen composition of the present invention for oral administration. As shown in Fig. 1, when the fatty acid ester of sucrose is mixed with the influenza vaccine especially at the weight ratio of 500 or more, the average particle diameter of the immunogen in the influenza vaccine increases to about 600nm, while the initial average particle diameter thereof is about 300nm. This increase in average particle diameter of the immunogen due to the presence of the fatty acid ester of sucrose is considered to contribute to the effect seen in the present invention. Also it is considered that, when the immunogen composition of the present invention is orally administered, the powdery immunogen is dissolved as the enteric coating of the composition is dissolved in the intestine and therefore the immunogen and the fatty acid ester of sucrose become in mixture state with efficient production of the effect of the immunogen.

The immunogen composition of the present invention, the process for preparation thereof, the effect thereof and examples of preparations will hereinafter be explained in detail.

### Example 1 (Preparation of powdery influenza vaccine)

One gram (1g) of sucrose laurate (trade name: L-1695 produced by Mitsubishi Kasei Shokuhin Kabushiki Kaisha, Japan) was dissolved in 10ml of phosphate buffer containing influenza HA vaccine (A/Kishu/X-34 strain, the same in the following examples) (the concentration of the vaccine protein being 50µg/ml). The solution was freeze-dried to produce about 1g of a powdery influenza vaccine.

### Example 2 (Preparation of powdery influenza vaccines)

Zero point five grams (0.5g) of sucrose palmitate (trade name: P-1670 produced by Mitsubishi Kasei Shokuhin Kabushiki Kaisha), 0.5g of sucrose stearate (trade name: S-1670 produced by Mitsubishi Kasei Shokuhin Kabushiki Kaisha) and 0.5g of sucrose oleate (trade name: O-1570 produced by Mitsubishi Kasei Shokuhin Kabushiki Kaisha) were each dissolved in 10ml of phosphate buffer containing influenza HA vaccine (the concentration of the vaccine protein being 50µg/ml). The solutions were freeze-dried to produce powdery influenza vaccines.

### Example 3 (Preparation of powdery influenza vaccine with 2 or more fatty acid esters of sucrose mixed)

Zero point two five grams (0.25g) of sucrose laurate (trade name: L-1695 produced by Mitsubishi Kasei Shokuhin Kabushiki Kaisha) and 0.25g of sucrose caprate (trade name: CT-1695 produced by Mitsubishi Kasei Shokuhin Kabushiki Kaisha) were dissolved in 10ml of phosphate buffer containing influenza HA vaccine (the concentration of the vaccine protein being 50µg/ml) (sucrose laurate and sucrose caprate being mixed in equal amount). The solution was freeze-dried to produce about 0.5g of a powdery influenza vaccine.

### Example 4 (Preparation of powdery influenza vaccine with 2 or more fatty acid esters of sucrose mixed)

Zero point two five grams (0.25g) of sucrose laurate (trade name: L-1695 produced by Mitsubishi Kasei Shokuhin Kabushiki Kaisha) and 0.25g of sucrose oleate (trade name: O-1570 produced by Mitsubishi Kasei Shokuhin Kabushiki Kaisha) were dissolved in 10ml of phosphate buffer containing influenza HA vaccine (the concentration of the vaccine protein being 50µg/ml) (sucrose laurate and sucrose caprate being mixed in equal amount). The solution was freeze-dried to produce about 0.5g of a powdery influenza vaccine.

### Example 5 (Preparation of vaccines using other immunogens)

Zero point five milligrams (0.5mg) of sucrose laurate (trade name: L-1695 produced by Mitsubishi Kasei Shokuhin Kabushiki Kaisha) were dissolved in 10ml of phosphate buffers, one containing influenza inactivated vaccine (A/Yamagata/120/86 strain), dry BCG vaccine or inactivated rotavirus antigen (the concentration of the vaccine protein being 50µg/ml each). The solutions were freeze-dried to produce powdery vaccines.

### Example 6

The powdery vaccine obtained in Example 1 was dissolved in PBS and adjusted to have a vaccine concentration of 50µg/ml. One milliliter (1ml) of this vaccine solution was orally administered to ddY mice (6 weeks, female, around 27g in weight, the same in the following examples) using an oral sound. Blood samples were taken three weeks after the administration, antibody titer therein was evaluated by HI (Hemagglutination inhibition) assay (Sakae Inoue, "Virus Experiments" published by Maruzen, p. 217). As control, phosphate buffer containing influenza HA vaccine (the concentration of the vaccine protein being 50µg/ml) was administered to mice under the same condition to evaluate sera thereof. The results are shown in Table 1.

**Table 1**

| Sample | Sucrose Laurate (mg) | Amount of Antigen (µg) | Antibody Valence in blood 3 weeks after immunization (HI value) |
|---|---|---|---|
| Vaccine of Example 1 | 100 | 50 | 512 |
| Control Vaccine Solution | - | 50 | 32 |

Generally, the infection of influenza has been reported to be inhibited when the antibody titer in blood obtained by influenza vaccine is 128 or more in HI value (Akira Otani, "Vaccines" published by Kodansha, p. 29). Therefore, Table 1 shows that the vaccine composition can provide a sufficient antibody titer at so practical a dosage as 50µg in terms of the antigen and, besides, by a single immunization.

### Example 7 (In case of using 50mg of fatty acid ester of sucrose)

Zero point five grams (0.5g) of sucrose laurate (trade name: L-1695 produced by Mitsubishi Kasei Shokuhin Kabushiki Kaisha) was dissolved in 10ml of phosphate buffers containing influenza HA vaccine at different concentrations of the vaccine protein, 100µg, 50µg, 25µg, 12.5µg and 6.25µg per milliliter. The solutions were freeze-dried to produce powdery influenza vaccines which had different weight ratios of sucrose laurate with respect to 1 of the vaccine protein, i.e., 500, 1,000, 2,000, 4,000 and 8,000.

The powdery vaccines thus obtained were each dissolved in 10ml of PBS. One milliliter (1ml) of each of these vaccine solutions was orally administered to ddY mice using an oral sound. Three weeks after the oral administration, a second oral administration was carried out to each group (one group consisting of 10 mice) under the same conditions as in the first administration. Blood samples were taken five weeks after the first administration, and the antibody titer therein was evaluated as described in the above example. The results are shown in Table 2.

**Table 2**

| Weight ratio of sucrose laurate with respect to vaccine protein | Sucrose Laurate (mg) | Amount of Antigen (µg) | Antibody Valence in blood 5 weeks after immunization (HI value) |
|---|---|---|---|
| 500 | 50 | 100 | 1024 |
| 1,000 | 50 | 50 | 512 |
| 2,000 | 50 | 25 | 1024 |
| 4,000 | 50 | 12.5 | 512 |
| 8,000 | 50 | 6.25 | 256 |

Table 2 shows that, when the mixed amount of the fatty acid ester of sucrose is constant, i.e., 50mg, the compositions can exhibit sufficient effectiveness providing the weight ratio of the fatty acid ester of sucrose with respect to 1 of the vaccine protein is 8,000 or less. Further, since the antibody titer generally tends to increase with increase of the amount of the antibody, high antibody titer is also expected when the amount of the antigen is 200µg (the weight ratio of the fatty acid ester of sucrose with respect to 1 of the vaccine protein being 250).

### Example 8

Zero point five grams (0.5g), 0.25g and 0.125g of sucrose laurate (trade name: L-1695 produced by Mitsubishi Kasei Shokuhin Kabushiki Kaisha) were each dissolved in 10ml of phosphate buffers containing influenza HA vaccine (the concentration of the vaccine protein being 50µg/ml). The solutions were freeze-dried to produce powdery influenza vaccines which had different weight ratios of sucrose laurate 1,000, 500, 250 with respect to 1 of the vaccine protein.

The powdery vaccines thus obtained were each dissolved in PBS and adjusted to have a vaccine concentration of 50µg/ml. The vaccine solutions were evaluated as described in Example 6. The results are shown in Table 3.

**Table 3**

| Weight ratio of sucrose laurate with respect to 1 of vaccine protein | Sucrose Laurate (mg) | Amount of Antigen (µg) | Antibody Valence in blood 3 weeks after immunization (HI value) |
|---|---|---|---|
| 1,000 | 50 | 50 | 512 |
| 500 | 25 | 50 | 128 |
| 250 | 12.5 | 50 | 64 |

Table 3 shows that as the weight ratio of the fatty acid ester of sucrose with respect to 1 of the vaccine protein becomes smaller, the antibody titer declines proportionally, and that the effectiveness of the composition cannot be obtained by a single immunization under the conditions of this example when the weight ratio becomes 250 or less.

### Example 9

The powdery vaccine obtained in Example 1 was dissolved in PBS and adjusted to have a vaccine concentration of 25µg/ml. This solution is orally administered to ddY mice as described in the above examples and blood samples were evaluated. The results are shown in Table 4.

**Table 4**

| Sample | Sucrose Laurate (mg) | Amount of Antigen (µg) | Antibody Valence in blood 3 weeks after immunization (HI value) |
|---|---|---|---|
| Vaccine of Example 1 | 100 | 25 | 256 |

Table 4 shows that sufficient antibody titer can be obtained by a single administration of 25µg of the antigen.

### Example 10 (Varying the weight ratio of fatty acid ester of sucrose to immunogen using 25mg of fatty acid ester of sucrose)

Zero point two five grams (0.25g) of sucrose laurate (trade name: L-1695 produced by Mitsubishi Kasei Shokuhin Kabushiki Kaisha) were dissolved in 10ml of phosphate buffers containing influenza HA vaccine at different concentrations of the vaccine protein, 100µg, 50µg, 25µg and 12.5µg per milliliter. The solutions were freeze-dried to produce powdery influenza vaccines which had different weight ratios of sucrose laurate 250, 500, 1,000 and 2,000 with respect to 1 of the vaccine protein.

The powdery vaccines thus obtained were each dissolved in 10ml of PBS. One milliliter (1ml) of each of these vaccine solutions was orally administered to ddY mice using an oral sound. Three weeks after the oral administration, a second oral administration was carried out to each group under the same conditions as in the first administration. Blood samples were taken five weeks after the first administration as described above. The results are shown in Table 5.

**Table 5**

| Weight ratio of sucrose laurate with respect to 1 of vaccine protein | Sucrose Laurate (mg) | Amount of Antigen (µg) | Antibody Valence in blood 5 weeks after immunization (HI value) |
|---|---|---|---|
| 250 | 25 | 100 | 512 |
| 500 | 25 | 50 | 256 |
| 1,000 | 25 | 25 | 128 |
| 2,000 | 25 | 12.5 | 256 |

Table 5 shows that, when the mixed amount of the fatty acid ester of sucrose is constant, i.e., 25mg, the compositions can exhibit sufficient effectiveness providing the weight ratio of the fatty acid ester of sucrose with respect to 1 of the vaccine protein is 250 to 2,000. Further, compared with the compositions with 50mg of the fatty acid ester of sucrose added (Example 7), it is apparent that, when the amount of the antigen is the same, the compositions with 50mg of the fatty acid ester of sucrose had better effect than the compositions with 25mg of the fatty acid ester of sucrose.

### Example 11 (Test on safety of powdery vaccine using mice)

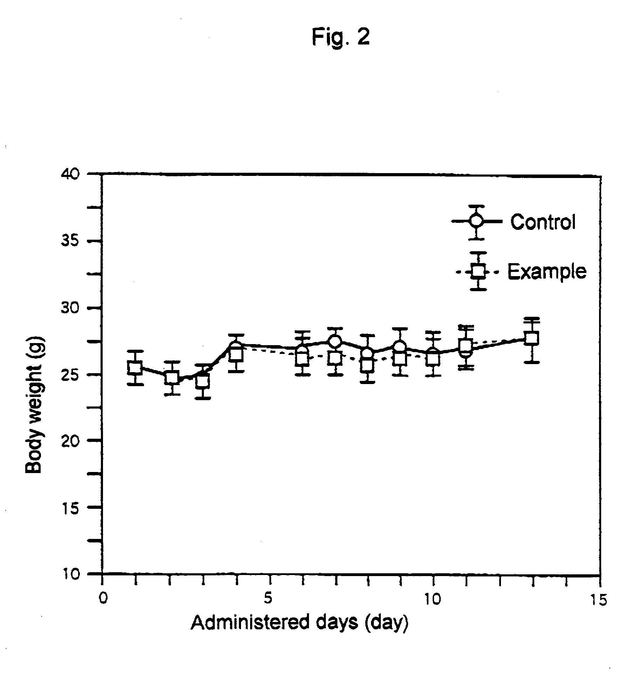

The powdery vaccine obtained in Example 1 was dissolved in PBS and adjusted to have a vaccine concentration of 50µg/ml. One milliliter (1ml) of this vaccine solution was orally administered to ddY mice using an oral sound. The same administration was repeated every day for the following two weeks while change in the weights of the mice and their status were observed to examine whether or not the powdery vaccine of the invention has toxicity. The results are shown in Figure 2.

Figure 2 shows the change in the weights of the mice to which the immunogen composition for oral administration of the present invention was administered every day for two weeks. The figure obviously shows that no change in weight was observed in the mice given the powdery vaccine obtained in Example 1 just as no change in weight were observed in a control group given only phosphoric acid buffered physiological salt solution every day for two weeks. No unusual cases such as death of mice or piloerection were observed, either.

### Example 12 (Test on effectiveness of powdery vaccine using monkeys)

Zero point five grams (0.5g) of sucrose laurate (trade name: L-1695 produced by Mitsubishi Kasei Shokuhin Kabushiki Kaisha) were dissolved in 10ml of phosphate buffer containing influenza HA vaccine (the concentration of the vaccine protein being 30µg/ml). The solution was freeze-dried to produce a powdery influenza vaccine.

The obtained powdery vaccine was dissolved in PBS and adjusted to have a vaccine concentration of 150µg/5ml. Five milliliters (5ml) of this vaccine solution was administered directly into the duodenum of cynomolgus monkey (male three or more years old, weighing 3.5kg, from the Philippines) using a fiberscope. Blood samples were taken three weeks after the administration, and the antibody titer therein was evaluated by HI value as described above. The results are shown in Table 6.

**Table 6**

| Sample | Sucrose Laurate (mg) | Amount of Antigen (µg) | Antibody Valence in blood 3 weeks after immunization (HI value) |
|---|---|---|---|
| Vaccine of Example 1 | 250 | 150 | 512 |

Table 6 shows that the composition can provide a sufficient antibody titer in the cynomolgus monkey weighing 100 or more times as much as mice at a dosage three times as much, 150µg, in terms of antigen.

### Example 13

One milliliter of the solution of the powdery influenza vaccine obtained in Example 3 with the two kinds of fatty acid esters of sucrose mixed was orally administered to ddY mice using an oral sound. The evaluation of immune response after the administration was performed as described above. The results are shown in Table 7.

**Table 7**

| Sample | Amount of Antigen | Antibody Valence in blood 3 weeks after immunization (HI value) |
|---|---|---|
| Sucrose laurate & sucrose caprate mixed in equal amount | 50µg | 128 |

Table 7 shows that the immunogen composition with Sucrose laurate and sucrose caprate mixed in equal amount exhibits effectiveness.

### Example 14 (Relation between the effectiveness and the number of carbons of the fatty acid of the fatty acid ester of sucrose)

Zero point five grams (0.5g) of sucrose caprylate (trade name: CE-1695 produced by Mitsubishi Kasei Shokuhin Kabushiki Kaisha), sucrose caprate (trade name: CT-1695 produced by Mitsubishi Kasei Shokuhin Kabushiki Kaisha), sucrose laurate (trade name: L-1695 produced by Mitsubishi Kasei Shokuhin Kabushiki Kaisha) and sucrose myristate (trade name: M-1695 produced by Mitsubishi Kasei Shokuhin Kabushiki Kaisha) were each dissolved as the fatty acid esters of sucrose in 10ml of phosphate buffer containing influenza HA vaccine (the concentration of the vaccine protein being 50µg/ml). One milliliter of the obtained solutions were each orally administered to ddY mice by an oral sound. Three weeks after the administration, blood samples were taken, and then second oral administration was given to each group under the same conditions as in the first administration. Two weeks after the second administration, i.e., five weeks after the first administration, blood samples were taken again. Antibody valences therein three and five weeks after the first administration are shown in Table 8.

**Table 8**

| Sample | Amount of Antigen | Antibody Valence in blood after immunization (HI value) | |
|---|---|---|---|
| | | 3 weeks | 5 weeks |
| Sucrose caprate | 50µg | 32 | 256 |
| Sucrose caprate | 50µg | 64 | 256 |
| Sucrose laurate | 50µg | 256 | 512 |
| Sucrose myristate | 50µg | 128 | 256 |

Table 8 shows that the immunogen compositions with sucrose myristate, sucrose laurate , sucrose caprate, and sucrose caprylate have effectiveness, with sucrose laurate taking a leading part.

### Example 15 (Improvement in preparation by adding lactose)

Since the fatty acid esters of sucrose have high moisture absorption, the fatty acid esters of sucrose sometimes cause problems such as poor fluidity in workability at preparation. Addition of lactose as an additive improved the workability and quickened dissolution in water. Further, the addition of lactose did not affect the effectiveness of the vaccine.

Zero point five grams (0.5g) of sucrose laurate (trade name: L-1695 produced by Mitsubishi Kasei Shokuhin Kabushiki Kaisha) and a mixture of 0.5g sucrose laurate (trade name: L-1695 produced by Mitsubishi Kasei Shokuhin Kabushiki Kaisha) and 0.5g of β-lactose anhydride (produced by DMV) were each dissolved in 10ml of phosphate buffer containing influenza HA vaccine (the concentration of the vaccine protein being 50µg/ml) and freeze-dried to produce powdery vaccines.

The obtained vaccines were each tested on solubility. The solubility test was performed in accordance with the dissolution test No. 2 (puddle method) of the Pharmacopoeia of Japan Twelfth Edition at a puddle rotate rate of 100rpm using 900ml of the disintegration test liquid No. 2 of the Pharmacopoeia of Japan Twelfth Edition at 37°C. Ninety milligrams (90mg) of the powdery vaccines with and without lactose were each formed in cylinder, and the time period in which each of the cylinders was completely dissolved was determined. The results are shown in Table 9.

**Table 9**

| Sample | Time required for dissolution in the disintegration test liquid No. 2 (minutes) |
|---|---|
| Powder vaccine without lactose | 30 |
| Powder vaccine with lactose added | 15 |

Table 9 shows that the addition of lactose shortened the time required for the dissolution of the powdery vaccines.

### Example 16 (Effect of addition of lactose on antibody titer)

Zero point five grams (0.5g) of sucrose laurate (trade name: L-1695 produced by Mitsubishi Kasei Shokuhin Kabushiki Kaisha) and a mixture of 0.5g sucrose laurate (trade name: L-1695 produced by Mitsubishi Kasei Shokuhin Kabushiki Kaisha) and 1.5g of β-lactose anhydride (produced by DMV) were each dissolved in 10ml of phosphate buffer containing influenza HA vaccine (the concentration of the vaccine protein being 50µg/ml) and freeze-dried to produce powdery vaccines. The obtained powdery vaccines were each dissolved in PBS and adjusted to have a vaccine concentration of 50µg/ml. One milliliter (1ml) of each of these vaccine solutions were each orally administered to ddY mice using an oral sound. Three weeks after the administration, blood samples were taken. The antibody titer therein was represented as HI value. The results are shown in Table 10.

**Table 10**

| Sample | Lactose | Sucrose Laurate | Amount of Antigen | Antibody valence in blood 3 weeks after immunization (HI value) |
|---|---|---|---|---|
| Powder vaccine without lactose | 0.15g | 0.05g | 50µg | 256 |
| Powder vaccine with lactose | - | 0.05g | 50µg | 256 |

Table 10 shows that the addition of lactose does not affect the antibody titer in blood.

### Preparation Example 1

The powdery influenza vaccine obtained in Example 1 was coated with an enteric film by a roll-stirring fluidized granulator (SPIR-A-FLOW produced by Freund's Sangyosha). Table 11 shows a recipe of enteric coating liquid used for the coating.

**Table 11**

| | |
|---|---|
| Hydroxypropyl methyl cellulose acetate succinate (HPMC-AS) | 5.2 parts by weight |
| Acetylated monoglyceride | 0.5 parts by weight |
| Methylene chloride | 47.2 parts by weight |
| Ethanol | 47.1 parts by weight |

The usage amount of the enteric coating liquid was set so that the amount of HPMC-AS therein was about 15 wt% with respect to the powdery influenza vaccine. The obtained enteric powdery influenza vaccine preparation stood the disintegration test for enteric preparations of the Pharmacopoeia of Japan.

### Preparation Example 2

The powdery influenza vaccine obtained in Example 1 was enclosed in hard gelatin capsules and provided with enteric coating by PAN coater (HI-CORTER. 48N type produced by Fruend's Sangyosha). The recipe of enteric coating liquid was the same as described in Preparation Example 2.

### Comparative Example 1

One gram of powdery influenza vaccines were obtained in the same way as described in Example 1 except that 1g of polyethylene glycol monostearate (MYS-40 produced by Nikko Chemical Kabushiki Kaisha, Japan) and 1g of polyoxyethylene·polyoxypropylene glycol (pluronic F-68 produced by Asahi Denka Kogyo Kabushiki Kaisha, Japan), both being nonionic surfactants, were used instead of sucrose laurate.

The obtained powdery vaccines were dissolved in PBS and adjusted to have a vaccine concentration of 50µg/ml. One milliliter (1ml) of these vaccine solutions were orally administered to ddY mice using an oral sound. Blood samples were taken three weeks after the administration, and the antibody titer therein was evaluated as HI value. The results are shown in Table 12.

**Table 12**

| Sample | Amount of Antigen | Antibody valence in blood 3 weeks after immunization (HI value) |
|---|---|---|
| Powder vaccine (MYS-40) | 50µg | 32 |
| Powder vaccine (pluronic F-68) | 50µg | 32 |

As clearly shown in Table 12, although both polyethylene glycol monostearate and polyoxyethylene·polyoxypropylene glycol are nonionic surfactants like sucrose laurate, the desired vaccinal effect cannot be achieved.

### Comparative Example 2

Instead of sucrose laurate used in Example 1, 1g of sodium lauryl sulfate (trade name: SLS produced by Nikko Chemical Kabushiki Kaisha) and dioctyl sulfosuccinate (trade name: Rapisol produced by Nippon Yushi, Japan) as anionic sulfactants, benzethonium chloride (trade name: HIAMINE produced by Sankyo Kabushiki Kaisha, Japan) and benzalkonium chloride (trade name: M2-100 produced by Nippon Yushi) as cationic surfactants were each dissolved in 10ml of phosphate buffer containing influenza HA vaccine (the vaccine protein concentration being 50µg/ml) as described in Example 1. The solutions were each freeze-dried to produce about 1g of powdery influenza vaccines.

The obtained powdery vaccines were each dissolved in PBS and adjusted to have a vaccine concentration of 50µg/ml. One milliliter (1ml) of theses vaccine solutions were orally administered to ddY mice using an oral sound. After the administration, most mice in the administered groups developed diarrhea or died, and accordingly the experiment was stopped.

This fact shows that the use of anionic and cationic surfactants is not preferable in light of safety.

### Comparative Example 3 (Comparison with microcapsules)

Microcapsules have been studied in the art as a carrier for orally administered vaccine antigens. For an example of microcapsule preparation, polylactic acid microcapsules were produced by a conventional method and compared with the present invention.

First, polyvinyl alcohol (produced by Unitika Ltd., Japan) was added to phosphate buffer containing influenza HA vaccine (the vaccine protein concentration being 100µg/ml) to produce 0.5% solution as an aqueous phase. Then, 12.5mg of polylactic acid was added to 10ml of methylene chloride as an oil phase. Zero point 5 milliliters (0.5ml) of the aqueous phase and 10ml of the oil phase were subjected to primary emulsification by a probe ultrasonic homogenizer (trade name: US-150T produced by Nippon Seiki, Japan) at 300W for 5 minutes. As an outer aqueous phase, polyvinyl alcohol (produced Kuraray Co., Ltd., Japan) was added to 250ml of 0.3M salt solution to make 2% solution. The outer aqueous phase was added to the primary emulsion, which was then subjected to secondary emulsification by a high-speed agitator (trade name: Polytrone PT-3000 produced by Kinematica-AG) at 10,000rpm for 5 minutes. The secondary emulsion was stirred with a stirrer to evaporate the solvent, then washed with water, and freeze-dried to produce a microcapsule preparation including influenza HA vaccine.

The obtained microcapsule preparation was dispersed in PBS and adjusted to have a vaccine concentration of 50µg/ml. One milliliter (1ml) of this dispersion was orally administered to ddY mice using an oral sound. Blood samples were taken three weeks after the administration, and the antibody titer therein was evaluated as described in the above examples. The results are shown in Table 13.

**Table 13**

| Sample | Amount of Antigen | Antibody valence in blood 3 weeks after immunization (HI value) |
|---|---|---|
| Microcapsule preparation | 50µg | <16 |
| Example 1 | 50µg | 512 |

Table 13 shows that no effectiveness is obtained with the microcapsule preparation on the same immune evaluation under the same conditions as the evaluation on the present invention. This proves that the art of the present invention is much more useful than the prior arts.

The present invention has realized an immunogen composition for oral administration safe and simple to administer which can provide the sufficient antibody titer by a single immunization with a practical dosage.

## Claims

1. An immunogen composition for oral administration comprising an immunogen and one, or two or more kinds of fatty acid esters of sucrose wherein the fatty acid has 6 to 18 carbons.

2. An immunogen composition according to claim 1 wherein the immunogen is influenza.

3. An immunogen composition according to claim 1 wherein the fatty acid ester of sucrose is sucrose laurate.

4. An immunogen composition according to any of the claims 1 to 3 wherein the weight ratio of the fatty acid ester of sucrose to the immunogen is 250 to 8,000 : 1.

5. An immunogen composition according to any of the claims 1 to 4 wherein the immunogen composition is made into an enteric preparation.

6. A process for preparing an immunogen composition for oral administration comprising mixing and dissolving a fatty acid ester of sucrose in a vaccine containing an immunogen at a weight ratio of 250 to 8,000 : 1 with respect to the immunogen, and then drying by dehydration to obtain a powdery vaccine.

7. A process for preparing an immunogen composition according to claim 6 wherein the powdery vaccine is made into an enteric preparation.
